(19) Europäisches Patentamt / European Patent Office / Office européen des brevets

(11) **EP 2 586 853 A1**

(12) **EUROPEAN PATENT APPLICATION**
published in accordance with Art. 153(4) EPC

(43) Date of publication:
01.05.2013 Bulletin 2013/18

(21) Application number: 11798272.8

(22) Date of filing: 24.06.2011

(51) Int Cl.:
$C10M\ 107/34$ (2006.01)    $C09K\ 5/04$ (2006.01)
$C10N\ 20/00$ (2006.01)    $C10N\ 20/02$ (2006.01)
$C10N\ 30/00$ (2006.01)

(86) International application number:
PCT/JP2011/064582

(87) International publication number:
WO 2011/162391 (29.12.2011 Gazette 2011/52)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 24.06.2010 JP 2010143542

(71) Applicant: Asahi Glass Company, Limited
Tokyo 100-8405 (JP)

(72) Inventors:
• ARAI, Takeaki
Tokyo 100-8405 (JP)

• NOMURA, Shingo
Tokyo 100-8405 (JP)
• SUZUKI, Chitoshi
Tokyo 100-8405 (JP)

(74) Representative: Blodig, Wolfgang
Wächtershäuser & Hartz
Patentanwaltspartnerschaft
Ottostrasse 4
80333 München (DE)

(54) **LUBRICATING OIL BASE OIL FOR HYDROCARBON REFRIGERANT AND LUBRICATING OIL COMPOSITION COMPRISING SAME**

(57) To provide a lubricant base oil for a refrigeration system, which has an appropriate viscosity and an appropriate compatibility with a hydrocarbon refrigerant. A lubricant base oil for a refrigerant consisting essentially of a $C_{1-8}$ hydrocarbon compound, wherein the lubricant base oil is a polyoxypropylene monohydrocarbyl ether having a number average molecular weight of from 500 to 3200, and the hydrocarbyl group has from 6 to 32 carbon atoms.

EP 2 586 853 A1

**Description**

TECHNICAL FIELD

The present invention relates to a lubricant base oil for a hydrocarbon refrigerant i.e. a lubricant base oil to be used for a refrigerant oil using a hydrocarbon refrigerant, and a lubricating oil composition comprising such a lubricant base oil. Particularly, it relates to a base oil having a polyoxypropylene monoether structure and having an appropriate compatibility with a hydrocarbon refrigerant, and a lubricating oil composition comprising such a base oil.

BACKGROUND ART

Heretofore, as a refrigerant for a compression refrigerator, a chlorofluorocarbon such as dichlorofluoromethane has been used in many cases. Such a chlorofluorocarbon is likely to destroy the ozone layer, and therefore, a study has been made on a hydrogen-containing chlorofluorocarbon compound. However, it is feared that a hydrogen-containing chlorofluorocarbon compound may present an influence over global warming.

Under the circumstances, it has been studied to use a hydrocarbon refrigerant free from such a problem. However, there are some problems when a hydrocarbon refrigerant is used.

The first problem relates to a lubricating oil for the refrigerant. Into a mineral oil or alkyl benzene which has been commonly used as a lubricating oil, a hydrocarbon refrigerant is dissolved, whereby the viscosity of the lubricating oil decreases to deteriorate the lubricating performance and at the same time the cooling power of the refrigerant decreases.

The second problem is that the hydrocarbon refrigerant is flammable. In order to minimize the risk at the time when the refrigerant leaks out, it is necessary to control the amount of the refrigerant to be sealed in a refrigerating system such as a refrigerator. However, as mentioned above, the refrigerant is likely to be dissolved in a lubricating oil, whereby the cooling power decreases, and it is difficult to reduce the amount.

In view of these problems, various lubricant base oils to be used for hydrocarbon refrigerants have been proposed. For example, a polyethylene glycol/polypropylene glycol copolymer having terminals sealed with a $C_{1-10}$ alkyl group (Patent Document 1), various polyalkylene glycol derivatives (Patent Document 2), and a polyethylene glycol/ polypropylene glycol copolymer having terminals sealed with a $C_{1-18}$ hydrocarbon group (Patent Document 3) have been proposed. Further, for a refrigerant mixture of ammonia and a hydrocarbon refrigerant, a polypropylene glycol monoether has been proposed (Patent Document 4).

PRIOR ART DOCUMENTS

PATENT DOCUMENTS

   Patent Document 1: JP-A-10-158671
   Patent Document 2: JP-A-11-349970
   Patent Document 3: JP-A-2005-290306
   Patent Document 4: JP-A-2000-96074

DISCLOSURE OF INVENTION

TECHNICAL PROBLEM

The above conventional lubricant base oils are improved in that the amounts to be dissolved in the refrigerants are reduced. However, they have a problem in oil return of the lubricant base oils i.e. the lubricating oil is separated and will not stay in a condenser. Therefore, it is an object of the present invention to provide a base oil which is free from such a problem and which has appropriate properties as a lubricant base oil and at the same time has an appropriate compatibility with a hydrocarbon refrigerant.

SOLUTION TO PROBLEM

The present inventors have found that with a lubricant base oil which is compatible with a refrigerant in a low temperature side region in a refrigeration system and which is separated from the refrigerant in a high temperature side region, it is possible to solve the above problem, to reduce abrasion in the system, to prevent leakage and to prolong the useful life of the system, and thus have accomplished the present invention. That is, the present invention provides a lubricant base oil for a refrigerant consisting essentially of a $C_{1-8}$ hydrocarbon compound, wherein the lubricant base oil is a polyoxypropylene monohydrocarbyl ether having a number average molecular weight of from 500 to 3200, and the hydrocarbyl group has from 6 to 32 carbon atoms, and a lubricating oil composition comprising such a lubricant base oil.

ADVANTAGEOUS EFFECTS OF INVENTION

The lubricant base oil of the present invention has an appropriate viscosity required for a lubricant base oil and at the same time has an appropriate compatibility with a hydrocarbon refrigerant, and it exhibits an excellent lubricating property and oil return property when used together with a hydrocarbon refrigerant and thus makes it possible to use the refrigeration system stably over a long period of time.

DESCRIPTION OF EMBODIMENTS

The lubricant base oil of the present invention (hereinafter sometimes referred to as "the base oil") has a polyoxypropylene main chain. By the after-described combination of the main chain having an appropriate polymerization degree and a hydrocarbyl group, it is possible to accomplish good oil return without dissolving the hydrocarbon solvent too much and to have an appropriate compatibility with the hydrocarbon solvent. Here, the hydrocarbyl group is a monovalent hydrocarbon group formed by removing one hydrogen atom from a hydrocarbon, and as described hereinafter, widely includes an alkyl group, a cycloalkyl group, an alkylene group, an aryl group, etc.

The base oil is a polyoxypropylene monohydrocarbyl ether, wherein the hydrocarbyl group has from 6 to 32 carbon atoms. One having a number of carbon atoms less than the above lower limit value has a low compatibility with the hydrocarbon refrigerant. On the other hand, one exceeding the above upper limit value will have a compatibility with the hydrocarbon refrigerant being too high, such being undesirable. The hydrocarbyl group may, for example, be a linear or branched alkyl group such as a hexyl group, a heptyl group, an octyl group, a nonyl group, a decyl group, an undecyl group, a dodecyl group, a tridecyl group, a tetradecyl group, a pentadecyl group, a hexadecyl group, an octadecyl group, a nonadecyl group, an eicosyl group, a docosyl group, a tetracosyl group, a 2-ethylhexyl group, a 2-ethyloctyl group, a 2-ethyldecyl group, a 2-hexyldecyl group, a 2-octyldecyl group, a 2-hexyldodecyl group or a 2-octyldodecyl group; a linear or branched alkenyl group such as an octenyl group, a decenyl group, an undecenyl group, a dodecenyl group, a tridecenyl group, a tetradecenyl group, a 2-ethyldecenyl group, a pentadecenyl group, a hexadecenyl group, a heptadecenyl group, an octadecenyl group, a nonadecenyloleyl group or a gadoleyl group; an alicyclic group such as a cyclohexyl group, a methylcyclohexyl group, an ethylcyclohexyl group or a dimethylcyclohexyl group; or an aromatic group such as a phenyl group, a methylphenyl group, an ethylphenyl group, a dimethylphenyl group, a nonylphenyl group, a dinonylphenyl group, a benzyl group or a methylbenzyl group. Among them, a $C_{6-24}$ alkyl group or a $C_{6-20}$ aryl group is preferred, and a $C_{8-18}$ alkyl group or a $C_{8-18}$ aryl group is more preferred.

The base oil may be a mixture of two or more polyoxypropylene monohydrocarbyl ethers having different groups among the above-mentioned groups.

The other terminal of the polyoxypropylene main chain is a hydroxy group, which may be a primary hydroxy group or a secondary hydroxy group.

The number average molecular weight of the base oil is from 500 to 3,200. If the number average molecular weight is less than the above lower limit value, the compatibility with the refrigerant tends to be too high, and if it exceeds the above upper limit value, the kinetic viscosity tends to be too high. Such a molecular weight range corresponds to a polymerization degree of oxypropylene units of from about 5 to about 50. The number average molecular weight is preferably from 700 to 2,500, more preferably from 800 to 1,400. Such a molecular weight may be measured by gel permeation chromatography (GPC) by using polystyrene as a standard sample.

In the base oil, the amount of high molecular weight impurities is preferably at most 5 mass%, more preferably at most 3 mass%, based on the total mass of the base oil. Such impurities are composed mainly of a dimer of the main component, particularly a diol, and are observed by GPC as a small peak in a high molecular weight range of from 1.8 to 2.2 times, of the main component peak. Here, the main component peak is a peak occupying at least 90% of the total peak area detected in the GPC measurement using a refractive index (RI) detector. Said high molecular weight range of from 1.8 to 2.2 times corresponds to from 900 to 7,040. Such high molecular weight impurities are inferior in the compatibility with the refrigerant and tend to increase the kinetic viscosity of the base oil and tend to lower the volume resistivity or the electrical insulating property of the base oil. The amount of such impurities can be obtained from the peak area ratio of the high molecular weight peak to the total peak area in the above GPC.

The base oil of the present invention may be obtained, for example, by reacting a desired hydrocarbyl alcohol with propylene oxide in the presence of a catalyst, e.g. an alkali catalyst such as potassium hydroxide, a composite metal cyanide complex catalyst or an acid catalyst. It is preferred that in the first step, the hydrocarbyl alcohol is reacted with an alkali catalyst to form an alcoholate, and water formed during the reaction is removed by reduced pressure treatment, followed by a reaction with propylene oxide in the second step. It is thereby possible to control the amount of impurities having a number average molecular weight exceeding 3,200 to be not more than the desired level. Further, it is also possible to control the amount of impurities having a number average molecular weight exceeding 3,200 by carrying out a step of removing methanol by using sodium methylate or potassium methylate as a catalyst.

The base oil is preferably such that the phase separation temperature on a high temperature side is from 50 to 80°C when mixed in a refrigerant/base oil ratio of 80/20 by a mass ratio of a refrigerant to the base oil in accordance with JIS K2211. If the phase separation temperature on a high temperature side is less than 50°C, the lubricating property tends to be low, and if it exceeds 80°C, the oil return tends to be problematic. The phase separation temperature on a high temperature side is preferably from 60 to 70°C.

Further, the base oil preferably has a kinetic viscosity of from 2 to 200 $mm^2$/s at 100°C. If the kinetic viscosity is less than the above lower limit value, the sealing property tends to be poor, and there will be a high risk of leakage of the refrigerant. On the other hand, if it exceeds the above upper limit value, the viscosity resistance tends to be high, whereby the lubricating property tends to be poor. The kinetic viscosity at 100°C is preferably from 5 to 100 $mm^2$/s. More preferably, it is about 10 $mm^2$/s in a case where the mass ratio of base oil/refrigerant is 20/80. Further, the base oil preferably has a volume resistivity of at least $1 \times 10^{10}$ $\Omega \cdot$cm, more preferably at least $6 \times 10^{10}$ $\Omega \cdot$cm, most preferably at least $1 \times 10^{11}$ $\Omega \cdot$cm. If the volume resistivity is less than $1 \times 10^{10}$ $\Omega \cdot$cm, the electrical insulating property may sometimes be deficient. The higher the volume resistivity, the better. However, it is preferably at most $1 \times 10^{15}$ $\Omega \cdot$cm, more preferably at most $1 \times 10^{14}$ $\Omega \cdot$cm. When the volume resistivity is at most $1 \times 10^{15}$ $\Omega \cdot$cm, the base oil or a composition containing the base oil is less likely to have static electricity, such being desirable from the viewpoint of safety.

The present invention relates also to a lubricating oil composition having various additives added to the base oil of the present invention in amounts not to impair the purpose of the present invention. Such additives may, for example, be an extreme pressure additive such as a phosphoric acid ester such as tricresyl phosphate (TCP), or a phosphorous acid ester such as trisnonylphenyl phosphite; a phenol type or amine type antioxidant; a stabilizer such as phenyl glycidyl ether, cyclohexene oxide or epoxidized soybean oil; a copper deactivator such as benzotriazole or its derivative; a defoaming agent such as silicone oil or fluorinated silicone oil; a load bearing additive, a chlorine scavenger, a cleaning dispersant, a viscosity index improver, an oiliness improver, a rust-preventive agent, a corrosion inhibitor, a pour point depressant, etc. The blend amount of these additives is usually from 0.5 to 10 mass%, based on the total mass of the base oil and the additives.

In the present invention, the refrigerant consists essentially of a hydrocarbon compound. Here, "essentially" means it consists solely of hydrocarbon except for impurities which are unavoidably contained. The hydrocarbon compound has from 1 to 8 carbon atoms, preferably from 1 to 5 carbon atoms, further preferably from 3 to 5 carbon atoms. If the number of carbon atoms exceeds the above upper limit value, the boiling point tends to be too high, such being undesirable as a refrigerant. The hydrocarbon compound may, for example, be methane, ethane, ethylene, propane, cyclopropane, propylene, n-butane, isobutene, n-pentane or isopentane. Propylene or propane is preferred. One type of these hydrocarbon compounds may be used alone, or two or more types may be used as mixed.

The amount of the base oil or the lubricating oil composition of the present invention to be used, may vary within such a wide range that the mass ratio of the hydrocarbon refrigerant/the base oil or lubricating oil composition is from 1/99 to 90/10, preferably from 10/90 to 50/50.

The base oil or the lubricating oil composition of the present invention is useful for various cooling systems including various hot-water supply systems, refrigerating/heating systems, gas heat pump systems, etc. for car air conditioners, room air conditioners, refrigerators, freezers, automatic vending machines or showcases.

EXAMPLES

Now, the present invention will be described in further detail with reference to Examples and Comparative Examples, but it should be understood that the present invention is by no means restricted by these Examples.

EXAMPLE 1

Into a 5 L autoclave, 520 g of 2-ethylhexyl alcohol (manufactured by KYOWA HAKKO CHEMICAL CO., LTD.) and 14.8 g of potassium hydroxide (purity: 95 mass%) as a catalyst were added, and reduced pressure treatment was carried out at 100°C for one hour to form an alcoholate while removing water. Then, the temperature of the autoclave was raised to 110°C, and 4,176 g of propylene oxide was introduced over a period of 10 hours. After confirming that the pressure in the autoclave became constant, and propylene oxide was all reacted, the content was withdrawn. To the content, synthetic magnesium silicate (Kyoward 600S, manufactured by Kyowa Chemical Industry Co., Ltd.) was added to have the catalyst adsorbed thereon, whereupon insolubles were removed by filtration. The obtained polyoxypropylene-2-ethylhexyl ether was found to have a number average molecular weight (Mn) of 1,170 by the GPC measurement, and the amount of impurities was 3 mass%. The details of the GPC measuring method will be described later.

EXAMPLE 2

A polyoxypropylene dodecyl ether having a number average molecular weight (Mn) of 1,000 was obtained in the same manner as in Example 1 except that 744 g of dodecyl alcohol (Kalcol 2098, manufactured by Kao Corporation) was used as a hydrocarbyl alcohol, 8.4 g of potassium hydroxide was added, reduced pressure treatment was carried out at 110°C for one hour, and thereafter, 3,248 g of propylene oxide was added. The amount of impurities was 2 mass%.

EXAMPLE 3

A polyoxypropyleneisotetradecyl ether having a number average molecular weight (Mn) of 1,190 was obtained in the same manner as in Example 1 except that 600 g of isotridecyl alcohol (Tridecanol, manufactured by KYOWA HAKKO CHEMICAL CO., LTD.) as a hydrocarbyl alcohol, 11.2 g of potassium hydroxide and 2,958 g of propylene oxide were used. The amount of impurities was 3 mass%.

EXAMPLE 4

A polyoxypropyleneoctadecyl ether having a number average molecular weight (Mn) of 1,550 was obtained in the same manner as in Example 1 except that 540 g of octadecyl alcohol (Kalcol 8098, manufactured by Kao Corporation) was used as a hydrocarbyl alcohol, 9.8 g of potassium hydroxide was added, reduced pressure treatment was carried out at 120°C for one hour, and thereafter, 2,552 g of propylene oxide was added. The amount of impurities was 4 mass%.

EXAMPLE 5

A polyoxypropylene-2-decyltetradecyl ether having a number average molecular weight (Mn) of 1,230 was obtained in the same manner as in Example 1 except that 708 g of 2-decyltetradecanol (Isofol 24, manufactured by Sasol Japan) as a hydrocarbyl alcohol and 7.7 g of potassium hydroxide were used, and 1,740 g of propylene oxide was introduced over a period of 8 hours. The amount of impurities was 2 mass%.

EXAMPLE 6

A polyoxypropylene oleyl ether having a number average molecular weight (Mn) of 1,780 was obtained in the same manner as in Example 1 except that 536 g of 9-octadecenyl alcohol (RIKACOL 90B, manufactured by New Japan Chemical Co., Ltd.) as a hydrocarbyl alcohol and 11.2 g of potassium hydroxide were used, and 3,016 g of propylene oxide was introduced over a period of 12 hours. The amount of impurities was 4 mass%.

EXAMPLE 7

A polyoxypropylene nonyl ether having a number average molecular weight of 570 was obtained in the same manner as in Example 1 except that 660 g of nonyl phenol (manufactured by Yokkaichi Chemical Company Limited) as a hydrocarbyl alcohol and 3.6 g of potassium hydroxide were used, and 1,044 g of propylene oxide was introduced over a period of 6 hours. The amount of impurities was 2 mass%.

EXAMPLE 8

Into a 5 L autoclave, 390 g of 2-ethylhexyl alcohol (manufactured by KYOWA HAKKO CHEMICAL CO., LTD.) and 12.9 g of potassium hydroxide (purity: 95 mass%) as a catalyst were added, and after raising the temperature to 110°C, 3,480 g of propylene oxide was introduced over a period of 10 hours. After the introduction of propylene oxide, it was confirmed that the pressure in the autoclave became constant, and propylene oxide was all reacted, whereupon the content was withdrawn. Then, in the same manner as in Example 1, the catalyst was removed to obtain a polyoxypropylene-2-ethylhexyl ether having a number average molecular weight (Mn) of 1,290. The amount of impurities was 8 mass%.

COMPARATIVE EXAMPLE 1

Into a 5 L autoclave, 216 g of powdery sodium methoxide was added, then the temperature was raised to 110°C, and 3,944 g of propylene oxide was introduced over a period of 10 hours. After the introduction of propylene oxide, it was confirmed that the pressure became constant, and propylene oxide was all reacted, whereupon the content was withdrawn. Thereafter, in the same manner as in Example 1, the catalyst was removed to obtain a polyoxypropylene methyl ether having a number average molecular weight (Mn) of 1,020. The amount of impurities was 4 mass%.

COMPARATIVE EXAMPLE 2

A polyoxypropylene methyl ether having a number average molecular weight (Mn) of 1,480 was obtained in the same manner as in Comparative Example 1 except that 162 g of powdery sodium methoxide was used, and 4,350 g of propylene oxide was introduced over a period of 12 hours. The amount of impurities was 4 mass%.

COMPARATIVE EXAMPLE 3

Into a 5 L autoclave, 222 g of n-butanol (manufactured by KYOWA HAKKO CHEMICAL CO., LTD.) and 0.3 g of a composite metal cyanide complex having a tert-butyl alcohol ligand, as a catalyst, were added. Thereafter, at 130°C, 200 g of propylene oxide was introduced over a period of two hours to induce an initial activity, and then, at 130°C, 3,976 g of propylene oxide was introduced over a period of 6 hours. Thereafter, it was confirmed that the pressure became constant, and propylene oxide was all reacted, whereupon the content was withdrawn to obtain a polyoxypropylene butyl ether having a number average molecular weight (Mn) of 1,470. The amount of impurities was 4 mass%.

COMPARATIVE EXAMPLE 4

Into a 5 L autoclave, 186 g of dodecyl alcohol (KALCOL 2098, manufactured by Kao Corporation) and 11.6 g of a potassium hydroxide (purity: 95 mass%) as a catalyst were added, and reduced pressure treatment was carried out at 110°C for one hour to form an alcoholate, while removing water. Thereafter, at 110°C, 3,480 g of propylene oxide was introduced over a period of 15 hours. It was confirmed that the pressure became constant, and propylene oxide was all reacted, whereupon the content was withdrawn. Then, in the same manner as in Example 1, the catalyst was removed to obtain a polyoxypropylene dodecyl ether having a number average molecular weight (Mn) of 3,670. The amount of impurities was 4 mass%.

COMPARATIVE EXAMPLE 5

Into a 5 L autoclave, 304 g of propylene glycol (manufactured by Asahi Glass Company, Limited) and 12.0 g of potassium hydroxide (purity: 95 mass%) as a catalyst were added, and reduced pressure treatment was carried out at 120°C for one hour to form an alcoholate, while removing water. Thereafter, at 110°C, 3,712 g of propylene oxide was introduced over a period of 12 hours. After the introduction, it was confirmed that the pressure became constant, and propylene oxide was all reacted, whereupon the content was withdrawn. Then, in the same manner as in Example 1, the catalyst was removed to obtain a polypropylene glycol having a number average molecular weight (Mn) of 930. High molecular weight impurities were not detected.

COMPARATIVE EXAMPLE 6

Into a 5 L autoclave, 600 g of isotridecanol (Tridecanol, manufactured by KYOWA HAKKO CHEMICAL CO., LTD.) and 2.7 g of a potassium hydroxide (purity: 95 mass%) as a catalyst were added, and reduced pressure treatment was carried out at 110°C for one hour to form an alcoholate, while removing water. Thereafter, at 110°C, 696 g of propylene oxide was introduced over a period of 4 hours. It was confirmed that the pressure became constant, and propylene oxide was all reacted, whereupon the content was withdrawn. Then, in the same manner as in Example 1, the catalyst was removed to obtain a polyoxypropyleneisotridecyl ether having a number average molecular weight (Mn) of 430. The amount of impurities was 1 mass%.

COMPARATIVE EXAMPLE 7

Into a 5 L autoclave, 540 g of octadecyl alcohol (KALCOL 8098, manufactured by Kao Corporation) and 10.2 g of a potassium hydroxide (purity: 95 mass%) as a catalyst were added, and reduced pressure treatment was carried out at 120°C for one hour to form an alcoholate, while removing water. Thereafter, at 110°C, 1,624 g of propylene oxide and 1,056 g of ethylene oxide were introduced over a period of 10 hours. It was confirmed that the pressure became constant, and propylene oxide was all reacted, whereupon the content was withdrawn. Then, in the same manner as in Example 1, the catalyst was removed to obtain a polyoxy(propylene/ethylene)octadecyl ether having a number average molecular weight (Mn) of 1,610. The amount of impurities was 3 mass%.

The obtained base oils were evaluated by the following methods. The results are shown in Tables 1 and 2.

<Kinetic viscosity>

Kinetic viscosities (mm$^2$/s) at temperatures of 40°C and 100°C were measured in accordance with JIS K2283-1983 by using a glass capillary viscometer.

<Compatibility with refrigerants>

Based on JIS K2211 "Test method for compatibility with refrigerant" of "refrigerant oil", a mixture comprising 20 mass% of a base oil and 80 mass% of a refrigerant (propane) was gradually cooled from room temperature to -60°C in a constant temperature tank, whereby the temperature at which the phase separation started, was measured by means of an optical sensor, and the mixture was gradually heated from room temperature to +90°C, whereby the temperature at which the phase separation started, was measured by means of an optical sensor. In the Tables, "partially compatible" means that a phase separation was observed under this condition (from - 60°C to 90°C), "completely compatible" means that the mixture was always dissolved, and "not compatible" means that the mixture was always phase-separated.

<Volume resistivity>

The volume resistivity (Ω·cm) of a base oil was measured, based on JIS C-2101 "test method for volume resistivity" of "electrically insulating oil".

<Molecular weight and amount of impurities>

The GPC measurement of a base oil was carried out under the following conditions, whereby the number average molecular weight (Mn) and the amount of impurities were obtained.

[Conditions for GPC measurement]

   Type of machine: HLC-8220GPC (manufactured by Tosoh Corporation)
   Data treating device: SC-8020 (manufactured by Tosoh Corporation)
   Column used: TSG gel G2500H (manufactured by Tosoh Corporation)
   Column temperature: 40°C, detector: RI, solvent: tetrahydrofuran, flow rate: 0.6 mL/min
   Sample concentration: 0.25 mass%, injected amount: 10 μL
   Standard sample for preparation of calibration curve: polystyrene ([Easical]PS-2 [Polystyrene Standards], manufactured by Polymer Laboratories)
   The mass % of impurities was calculated by the following formula.

$$\text{Mass \% of impurities} = \text{high molecular weight peak area} \times 100\ /\ \text{total peak area}$$

TABLE 1

| | Ex. 1 | Ex. 2 | Ex. 3 | Ex. 4 | Ex. 5 | Ex. 6 | Ex. 7 | Ex. 8 |
|---|---|---|---|---|---|---|---|---|
| Hydrocarbyl group | 2-Ethylhexyl | Dodecyl | Isotridecyl | Octadecyl | 2-Decyltetradecanyl | 9-Octadecenyl | Nonylphenyl | 2-Ethylhexyl |
| Main chain | Polypropylene oxide | Polypropylene oxide | Polypropylene oxide | Polypropylene oxide | Poly-propylene oxide | Polypropylene oxide | Polypropylene oxide | Polypropylene oxide |
| Number average molecular weight | 1170 | 1000 | 1190 | 1550 | 1230 | 1780 | 570 | 1290 |
| Impurities (mass%) | 3 | 2 | 3 | 4 | 2 | 4 | 2 | 8 |
| Compatibility with refrigerant | Partially compatible | Partially compatible | Partially compatible | Partially compatible | Partially compatible | Partially compatible | Partially compatible | Partially compatible |
| Phase separation temperature on high temperature side (°C) | 67 | 61 | 66 | 55 | 60 | 53 | 88 | 40 |
| Phase separation temperature on low temperature side (°C) | -60> | -60> | -40 | -40 | -35 | -40 | -60> | -30 |
| Kinetic viscosity 40°C (mm²/s) | 60.5 | 56.2 | 73.8 | 100.2 | 75.3 | 129.8 | 82 | 62.1 |
| Kinetic viscosity 100°C (mm²/s) | 11.3 | 10.2 | 13.5 | 17.0 | 13.1 | 21.1 | 8.5 | 11.8 |

(continued)

| | Ex. 1 | Ex. 2 | Ex. 3 | Ex. 4 | Ex. 5 | Ex. 6 | Ex. 7 | Ex. 8 |
|---|---|---|---|---|---|---|---|---|
| Volume resistivity ($\Omega \cdot cm$) | $1.2 \times 10^{11}$ | $9.2 \times 10^{10}$ | $1.0 \times 10^{11}$ | $4.0 \times 10^{11}$ | $5.0 \times 10^{11}$ | $4.0 \times 10^{11}$ | $6.5 \times 10^{10}$ | $9.4 \times 10^{10}$ |

TABLE 2

| | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|
| Hydrocarbyl group | Methyl | Methyl | Butyl | Dodecyl | - | Isotridecyl | Octadecyl |
| Main chain | Polypropylene oxide | Polypropylene oxide | Polypropylene oxide | Polypropylene oxide | Polypropylene oxide | Polypropylene oxide | Polypropylene oxide/polyethylene oxide |
| Number average molecular weight | 1020 | 1480 | 1470 | 3670 | 930 | 430 | 1610 |
| Impurities (mass%) | 4 | 4 | 4 | 4 | (none) | 1 | 3 |
| Compatibility with refrigerant | Partially compatible | Partially compatible | Partially compatible | Partially compatible | Not compatible | Completely compatible | Not compatible |
| Phase separation temperature on high temperature side (°C) | 60 | 30 | 40 | 30 | | 90< | |
| Phase separation temperature on low temperature side (°C) | -25 | -20 | -30 | -20 | | -60> | |
| Kinetic viscosity 40°C ($mm^2/s$) | 46.8 | 90.4 | 91.7 | 300 | 88.4 | 59.3 | 109.2 |
| Kinetic viscosity 100°C ($mm^2/s$) | 9.3 | 15.6 | 16.1 | 47.6 | 10.9 | 6.2 | 20.5 |
| Volume resistivity ($\Omega \cdot cm$) | $1 \times 10^{10}$> | $3.9 \times 10^{10}$ | $5.1 \times 10^{10}$ | $2.5 \times 10^{12}$ | $1.5 \times 10^{10}$ | $4.5 \times 10^{13}$ | $1.8 \times 10^{10}$ |

As shown in Table 1, the base oils in Examples were within a suitable range in each of the compatibility with the refrigerant, the kinetic viscosity and the volume resistivity. On the other hand, as shown in Table 2, one having no hydrocarbyl group (Comparative Example 5), one having a number of carbon atoms in the hydrocarbyl group being smaller than the range of the present invention (Comparative Examples 1 to 3) and one containing polyoxyethylene in the main chain (Comparative Example 7) were inferior in the compatibility with the refrigerant. Further, one having a molecular weight exceeding the range of the present invention (Comparative Example 4) had a high kinetic viscosity. On the other hand, one having the molecular weight being less than the range of the present invention (Comparative Example 6) had a compatibility with the refrigerant being too high. Thus, each one was not suitable as a lubricating oil.

INDUSTRIAL APPLICABILITY

The lubricant base oil or the lubricating oil composition of the present invention has an appropriate compatibility with a hydrocarbon refrigerant and is capable of being used stably over a long period of time for a cooling system such that it exhibits excellent lubricating property and oil return, and thus it can be used for various cooling systems including various hot-water supply systems, refrigerating/heating systems or gas heat pump systems, etc. for car air conditioners, room air conditioners, refrigerators, freezers, automatic vending machines or showcases.
The entire disclosure of Japanese Patent Application No. 2010-143542 filed on June 24, 2010 including specification, claims and summary is incorporated herein by reference in its entirety.

**Claims**

1.    A lubricant base oil for a refrigerant consisting essentially of a $C_{1-8}$ hydrocarbon compound, wherein the lubricant base oil is a polyoxypropylene monohydrocarbyl ether having a number average molecular weight of from 500 to 3200, and the hydrocarbyl group has from 6 to 32 carbon atoms.

2.    The lubricant base oil according to Claim 1, wherein the amount of impurities having a number average molecular weight of from 1.8 to 2.2 times the number average molecular weight of the polyoxypropylene monohydrocarbyl ether, contained in the lubricant base oil, is at most 5 mass%, based on the total amount of the lubricant base oil.

3.    The lubricant base oil according to Claim 1 or 2, of which the phase separation temperature on a high temperature side as measured in accordance with JIS K2211 is at least 50°C.

4.    The lubricant base oil according to any one of Claims 1 to 3, of which the kinetic viscosity at 100°C is from 2 to 200 $mm^2$/s.

5.    The lubricant base oil according to any one of Claims 1 to 4, of which the volume resistivity is at least $1\times10^{10}\Omega\cdot cm$ and at most $1\times10^{15}\Omega\cdot cm$.

6.    The lubricant base oil according to any one of Claims 1 to 5, wherein the hydrocarbyl group in the polyoxypropylene monohydrocarbyl ether contained in the lubricant base oil is a $C_{8-18}$ alkyl group or a $C_{8-18}$ aryl group.

7.    The lubricant base oil according to any one of Claims 1 to 6, wherein the refrigerant is propane.

8.    The lubricant base oil according to any one of Claims 1 to 6, wherein the refrigerant is propylene.

9.    A lubricating oil composition comprising the lubricant base oil as defined in any one of Claims 1 to 8.

10.   The lubricating oil composition according to Claim 9, which is used for a hot-water supply system, a refrigerating/heating system or a gas heat pump system for a car air conditioner, a room air conditioner, a refrigerator, a freezer, an automatic vending machine or a showcase.

<div align="center">

**INTERNATIONAL SEARCH REPORT**

</div>

| | International application No. |
|---|---|
| | PCT/JP2011/064582 |

**A. CLASSIFICATION OF SUBJECT MATTER**
*C10M107/34*(2006.01)i, *C09K5/04*(2006.01)i, *C10N20/00*(2006.01)n, *C10N20/02*
(2006.01)n, *C10N30/00*(2006.01)n

According to International Patent Classification (IPC) or to both national classification and IPC

**B. FIELDS SEARCHED**

Minimum documentation searched (classification system followed by classification symbols)
C10M107/34, C09K5/04, C10N20/00, C10N20/02, C10N30/00

Documentation searched other than minimum documentation to the extent that such documents are included in the fields searched
Jitsuyo Shinan Koho        1922–1996   Jitsuyo Shinan Toroku Koho   1996–2011
Kokai Jitsuyo Shinan Koho   1971–2011   Toroku Jitsuyo Shinan Koho   1994–2011

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2005-290306 A  (Idemitsu Kosan Co., Ltd.), 20 October 2005 (20.10.2005), claims; examples & US 2007/0164252 A1    & EP 1734101 A1 & WO 2005/095557 A1 | 1-10 |
| A | JP 11-349970 A  (Daikin Industries, Ltd.), 21 December 1999 (21.12.1999), claims; examples (Family: none) | 1-10 |
| A | JP 2004-43611 A  (Japan Energy Corp.), 12 February 2004 (12.02.2004), claims; examples & KR 10-2004-0007307 A   & CN 1472293 A | 1-10 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 September, 2011 (08.09.11) | 27 September, 2011 (27.09.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/064582

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2000-96074 A  (Nippon Mitsubishi Oil Corp.), 04 April 2000 (04.04.2000), claims; examples & US 6239086 B1          & EP 989180 A1 | 1-10 |

Form PCT/ISA/210 (continuation of second sheet) (July 2009)

**EP 2 586 853 A1**

**REFERENCES CITED IN THE DESCRIPTION**

**Patent documents cited in the description**

- JP 10158671 A
- JP 11349970 A
- JP 2005290306 A
- JP 2000096074 A
- JP 2010143542 A